# EUROPEAN PATENT SPECIFICATION

(11) **EP 4 338 718 B1**
(45) Date of publication and mention of the grant of the patent: **04.03.2026**
(21) Application number: 22195538.8
(22) Date of filing: 14.09.2022
(51) Int. Cl.: A61F 9/02

(54) **ROLL-OFF FILM SYSTEM FOR GOGGLES**
ABROLLBARE FOLIENANLAGE FÜR SCHUTZBRILLEN
SYSTÈME DE FILM À DÉROULEMENT POUR LUNETTES

(43) Date of publication of application: 20.03.2024
(73) Proprietor: Xiamen Anbo Sports Goods Co., Ltd., Xiamen, Fujian (CN)
(72) Inventor: CHEN, Jalon, Xiamen (CN); CHEN, James, Xiamen (CN); SHEN, Xiaoqiang, Xiamen (CN)
(74) Representative: Metida

(56) References cited:
- GB-A- 2 495 984
- US-A- 4 748 697
- US-A1- 2021 030 592
- US-B2- 9 839 558

## Description

### TECHNICAL FIELD

The present disclosure relates to a roll-off film system, and more particularly to a roll-off film system for goggles.

### BACKGROUND

The existing roll-off film system for goggles are complicated to assemble. The roll-off film needs to be assembled separately before being rolled onto two rotating shafts, then the roll-off films are assembled in canisters on both sides of the goggles, so the films are getting loose easily from the rotating shafts when assembled. The existing technology also has the following disadvantages.
(1) After the roll-off film is used up, it is difficult to replace the film, which not only takes time and effort, but also the user needs to learn how to disassemble and assemble the film.
(2) When the user replaces the roll-off film, if the whole system is replaced together, not only the cost is too high, but also it is difficult to disassemble.
(3) Due to the difficult assembly process, it is prone to assemble wrongly, and shorten the lifetime of the roll-off film system, or damage roll-off film system to cause the roll-off film system complete failure.

US publication No.US 748697 A discloses a face mask with a cylindrically-shaped canister provided at a side of the mask. The canister is further characterized by a cylindrical canister barrel, closed by a canister top and a canister bottom.

US publication No.US 9839558 B2 discloses a roll-off film system having a film dispensing canister and a film receiving canister. The film dispensing canister and the film receiving canister are both formed by a front casing and rear casing. The front casing of the film dispensing canister may include a coupling mechanism configured to couple the front casing to the rear casing. Coupling mechanisms each may include a deformable hook. The rear casings may include two lens attachment openings through which the lens attachment mechanism may be inserted. Tear-off pins may be disposed on front casing at which a user may pull to separate the front casing from the rear casing.

British publication No. GB 2495984 A discloses a goggle system having an actuating device. The actuating device is mounted to the body and located on two sides of the lens. the actuating device includes two reels respectively on two sides of the front face of the lens. Two ends of the transparent film are respectively attached to the reels. A button is provided on the reel and can be operated to move the transparent film.

US publication No. US 2021/030592 A1 discloses a mud shield configured to attach to a goggle frame, a goggle system includes a left canister and a right canister, where the film is conveyed from one of the left canister or the right canister to the other of the left canister or the right canister.

### SUMMARY

The objective of the present disclosure is to provide a roll-off film system to solve the disadvantages of the existing system proposed in the background art.

To achieve the above-mentioned objective, the present disclosure provides a roll-off film system for according to claim 1.

In an embodiment, the second canister is separably provided with a shaft; a straight slot is provided at a front end of the shaft; a front end of the second canister is movably mounted with a mechanism; and the front end of the shaft passes through the mechanism and extends to an inside thereof.

In an embodiment, a puller is movably connected to a right end of the mechanism.

In an embodiment, one end of the locker penetrates through the case to clamp with the connecting post, and the cover plate is configured to fix at a bottom of the case.

In an embodiment, the first canister and the second canister are provided with a respective guiding slot, both sides of the second inner cap and the first inner cap are fixedly connected with respective holding plates matching with the corresponding guiding slots.

Compared with the prior art, for roll-off film system for goggles, the first cap and the second cap are fixedly connected by the bridge, and the roll-off film and the shaft are fixed on the first and second caps to obtain a set of accessories. After the roll-off film is used up, the first and second caps with the roll-off film are removed, and a new set of accessories are mounted, and it can be used normally. Through the cooperation of various precision structures, users can replace the roll-off film more simply and efficiently. The whole set of accessories with the roll-off film can be easily assembled and disassembled. Even people who never uses the system can easily and efficiently replace it correctly. The roll-off film system can be prefabricated to satisfy quality standard without waste or damage.

### BRIEF DESCRIPTION OF THE DRAWINGS

FIG. 1 is a front view of a roll-off film system for goggles according to one embodiment of the present disclosure;
FIG. 2 is an exploded view of the roll-off film system for goggles according to one embodiment of the present disclosure;
FIG. 3 is a bottom view of a first cap, a second cap and a bridge according to one embodiment of the present disclosure;
FIG. 4 is a structural diagram showing a first canister according to one embodiment of the present disclosure;
FIG. 5 is a structural diagram showing a second canister according to one embodiment of the present disclosure;
FIG. 6 is a structural diagram showing a shaft according to one embodiment of the present disclosure;
FIG. 7 is a structural diagram showing a fixation system on a goggle according to one embodiment of the present disclosure;
FIG. 8 is an exploded view of the fixation system according to one embodiment of the present disclosure; and
FIG. 9 is a structural diagram showing a back of the first cap and the second cap according to one embodiment of the present disclosure.

In the Figures: 1, goggle; 2, roll off system; 3, first cap; 4, second cap; 5, bridge; 6, first canister; 7, second canister; 8, shaft; 9, mechanism; 10, puller; 11, hook; 12, first receiving groove; 13, second receiving groove; 14, fixation rib; 15, accommodating slot; 16, straight slot; 17, fixation system; 18, guiding slot; 19, cover plate; 20, locker; 21, connecting post; 22, fixation plate; 23, case; 24, holding plate; 25, first inner cap; 26, second inner cap.

### DETAILED DESCRIPTION OF EMBODIMENTS

The technical solution in the present disclosure will be described clearly and completely below with reference to the accompanying drawings and embodiments. Obviously, the described embodiments are only some, but not all embodiments of the present disclosure. Based on the disclosed embodiments of the present disclosure, all other embodiments obtained by those skilled in the art without creative efforts shall fall within protection scope of the present disclosure.

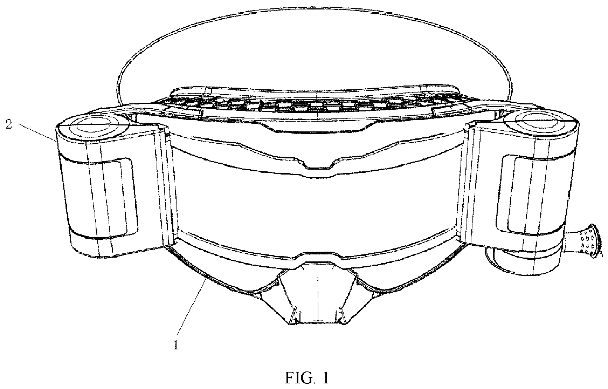

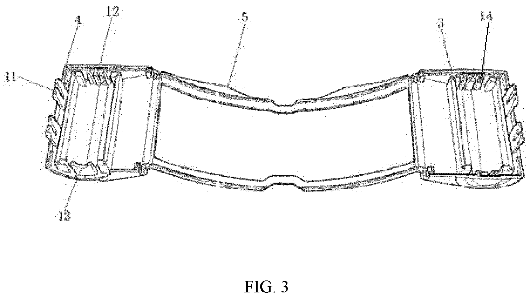

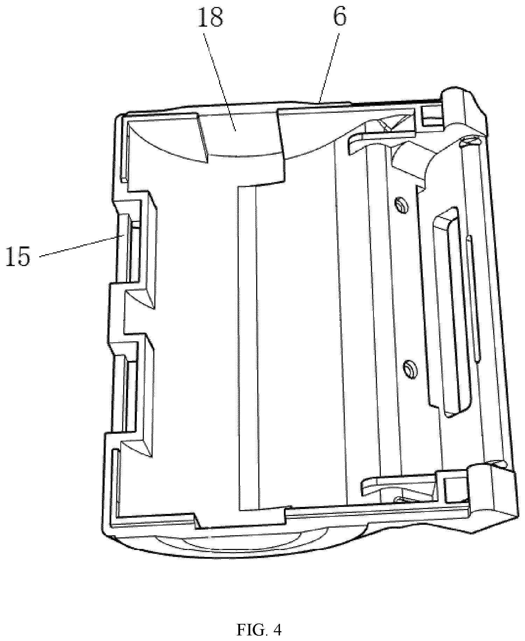

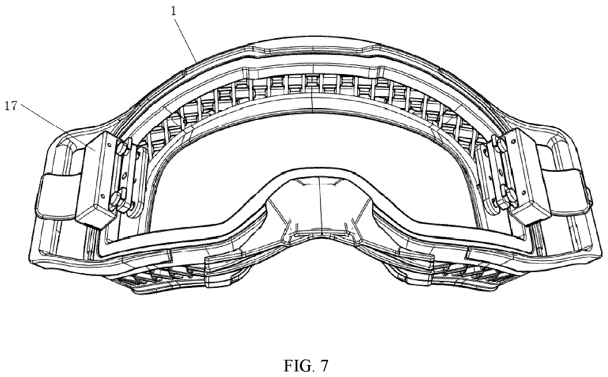

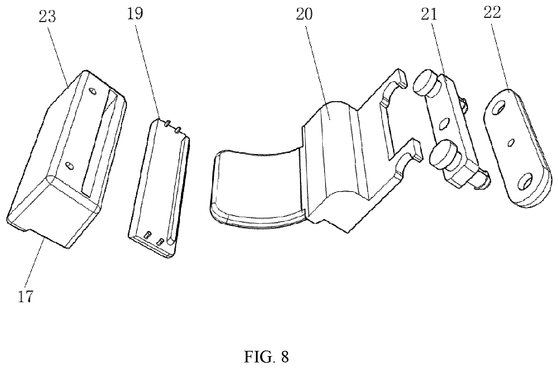

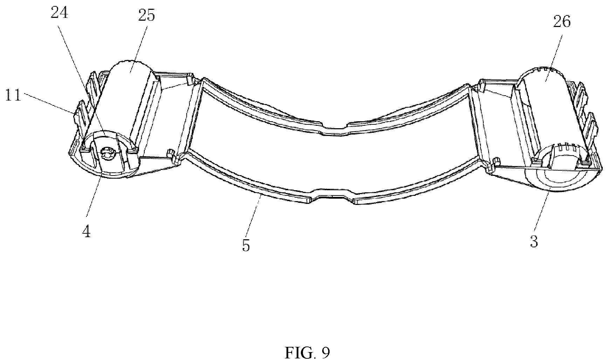

Referring to FIGS. 1-8, the present disclosure provides a roll-off film system for goggles which includes a goggle 1 and a roll off system 2. The roll off system 2 includes a first cap 3, a second cap 4, a bridge 5, a first canister 6, a second canister 7 and a fixation system 17. The first cap 3 and the second cap 4 are fixedly connected through the bridge 5, and the first canister 6 and the second canister 7 are respectively fixedly connected to the goggle 1 through the fixation system 17. Both ends of a bottom of the first cap 3 are respectively provided with the fixation ribs 14 which are used to prevent a roll-off film from getting loose. One end of a rotating shaft supporting the roll-off film is provided with holes matched with the fixation ribs 14. The fixation ribs 14 are matched with the holes to prevent the roll-off film from rotating in a reverse direction, and specifically, such a structure has a function similar to that of a ratchet and pawl mechanism. As shown in FIG. 3, ends of a bottom of the second cap 4 are respectively provided with a first receiving groove 12 anda second receiving groove 13. Hooks 11 are fixedly disposed on bottoms of sides of the first cap 3 and the second cap 4 away from the bridge 5. The sides of the first canister 6 and the second canister 7 away from the goggle 1 are provided with a respective accommodating slot 15. The accommodating slots 15 of the first canister 6 and the second canister 7 are matching with the corresponding hooks 11. The hooks 11 can be inserted into the corresponding accommodating slot 15 to realize connection between the first canister 6 and the first cap 3 and connection between the second canister 7 and the second cap 4. Front and rear sides of the first canister 6 and the second canister 7 are provided with a respective guiding slot 18. The guiding slots 18 are arranged for matching the holding plate 24 and the first receiving groove 12 for connection between the first canister 6 and the first cap 3 and connection between the second canister 7 and the second cap 4. The fixation system 17 includes a cover plate 19, a locker 20, a connecting post 21, a fixation plate 22 and a case 23. Specifically, the fixation plate 22 is fixedly connected to the goggle 1 through the connecting post 21. As shown in FIG. 9, a second inner cap 26 and a first inner cap 25 are respectively fixedly connected to bottoms of the first cap 3 and the second cap 4.Both sides of the second inner cap 26 and the first inner cap 25 are fixedly connected with holding plates 24 matching with the guiding slots 18.

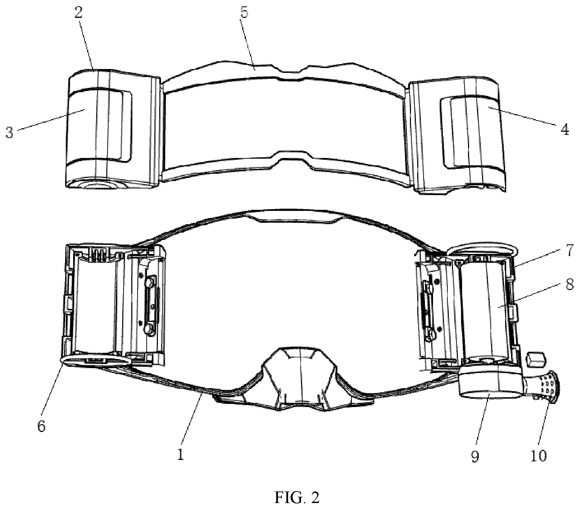

In FIG. 2, the first cap 3, the second cap 4 and the bridge 5 are arranged for mounting the roll-off film. When the goggle needs to be replaced with the roll-off film, the first and second caps with the roll-off film will be completely replaced. The systems on both sides of the goggle are connected by a bridge or in other means to ensure modularity and stability as well as simplicity in the process of replacing the roll-off film.

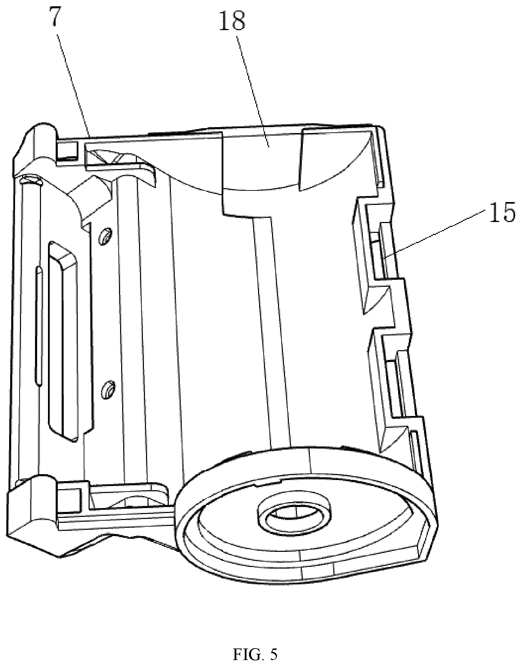

In FIG. 5, the first cap 3 and the second cap 4 are fixed by screws, but not limited to it. A roll-off film is provided on the shaft 8.

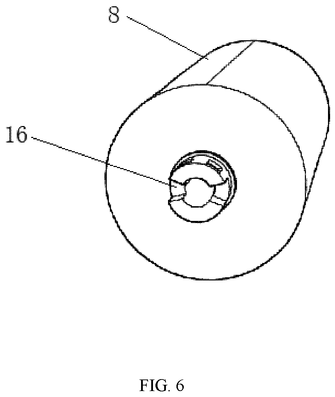

Referring to FIGS. 2 and 6 again, the second canister 7 is separably provided with a shaft 8. A straight slot 16 is opened at a front end of the shaft 8. The front end of the shaft 8 penetrates a mechanism 9 and extends to the inside thereof. A front end of the second canister 7 is movably mounted with a mechanism 9, and the straight slot 16 is used to cooperate with an output end of the mechanism 9 to replace the roll-off film.

A puller 10 is movably connected to a right end of the mechanism 9. By pulling the puller 10, the shaft 8 is rotated to replace the roll-off film. After the puller 10 is released, the puller 10 will be automatically reset under the action of an elastic member inside the mechanism 9. The collecting film method is the prior art, so it will not be repeated here.

One end of the locker 20 penetrates through the case 23 and is clamped with the connecting post 21, and the cover plate 19 is movably connected to the bottom of the case 23.

Thus, for the roll-off film system for goggles, the first cap and the second cap are fixedly connected by the bridge, the roll-off film and the shaft are fixed on the first and second caps to obtain a set of accessories. After the roll-off film is used up, the first and second caps with the roll-off film are directly removed, and a new set of accessories are mounted, and it can be used normally. Through the cooperation of various precision structures, users can replace the roll-off film more simply and efficiently. The whole set of accessories having the roll-off film can be easily assembled and disassembled. Even people who never use the system can easily and efficiently replace it correctly. The roll-off film system can be prefabricated to satisfy quality standard without waste or damage.

While the present disclosure has been described in detail with reference to the embodiments, it should be understood that it is still possible to modify the technical solutions in the foregoing embodiments, or perform replacements to some of the technical features. Understandably, any modifications and replacements made by those skilled in the art without departing from the spirit of the disclosure should fall within the scope of the disclosure defined by the present claims.

## Claims

1. A roll-off film system for goggles, comprising:
a goggle (1); and
a roll off system (2);
wherein the roll off system (2) comprises a first cap (3), a second cap (4), a first canister (6), a second canister (7) and a fixation system (17), and the first canister (6) and the second canister (7) are fixed on the goggle (1) through the fixation system (17),and sides of the first canister (6) and the second canister (7) away from the goggle (1) are provided with a respective accommodating slot (15) matching the corresponding hook (11);
**characterized in that** the roll off system(2) further comprises a bridge (5),the first cap (3) and the second cap (4) being fixedly connected by the bridge (5), both ends of a bottom of the first cap (3) being respectively provided with a fixation rib (14), the ends of a bottom of the second cap (4) being respectively provided with a first receiving groove (12) and a second receiving groove (13), and further comprising hooks (11) respectively disposed on bottoms of sides of the first cap (3) and the second cap (4) away from the bridge (5), and further comprising a second inner cap (26) and a first inner cap (25) respectively fixedly connected to bottoms of the first cap (3) and the second cap (4), the second canister (7) being separably provided with a shaft (8), a roll-off film and the shaft (8) being fixed on the first cap (3) and second cap (4) to obtain a whole set of accessories including the first cap (3)and second cap(4) with the roll-off film, the shaft (8),the bridge(5), the first inner cap(25),the second inner cap (26) to be replaced after the roll-off film is used up, and further **characterized in that**
the fixation system (17) comprises a cover plate (19), a locker (20), a connecting post (21), a fixation plate (22) and a case (23), and the fixation plate (22) is fixed on the goggle (1) through the connecting post (21).

2. The roll-off film system for goggles according to claim 1, wherein a straight slot (16) is provided at a front end of the shaft (8), a front end of the second canister (7) is movably mounted with a mechanism (9), and the front end of the shaft (8) passes through the mechanism (9) and extends to an inside thereof.

3. The roll-off film system for goggles according to claim 2, wherein a puller (10) is movably connected to a right end of the mechanism (9).

4. The roll-off film system for goggles according to claim 1, wherein one end of the locker (20) penetrates through the case (23) to clamp with the connecting post (21), and the cover plate (19) is configured to fix at a bottom of the case (23).

5. The roll-off film system for goggles according to claim 1, wherein the first canister (6) and the second canister (7) are provided with a respective guiding slot (18), both sides of the second inner cap (26) and the first inner cap (25) are fixedly connected with respective holding plates (24) matching with the corresponding guiding slots (18).

## Patentansprüche

1. Ein aufrollbares Foliensystem für Schutzbrillen, bestehend aus: Eine Schutzbrille (1); und Roll-on/Roll-off-System (2). Das Rollsystem (2) umfasst eine erste Abdeckung (3), eine zweite Abdeckung (4), einen ersten Behälter (6), einen zweiten Behälter (7) und ein Befestigungssystem (17). Der erste Behälter (6) und der zweite Behälter (7) werden mittels des Befestigungssystems (17) an der Schutzbrille (1) befestigt. Die von der Schutzbrille (1) abgewandten Seiten des ersten Behälters (6) und des zweiten Behälters (7) weisen Aufnahmenuten (15) auf, die zu den entsprechenden Haken (11) passen. Das Wickelsystem (2) zeichnet sich dadurch aus, dass es ferner eine Brücke (5) umfasst, die erste Abdeckung (3) und die zweite Abdeckung (4) durch die Brücke (5) fest miteinander verbunden sind, die unteren Enden der ersten Abdeckung (3) jeweils mit Befestigungsrippen (14) versehen sind, die unteren Enden der zweiten Abdeckung (4) jeweils mit einer ersten Aufnahmenut (12) und einer zweiten Aufnahmenut (13) versehen sind, die Unterseiten der ersten Abdeckung (3) und der zweiten Abdeckung (4), die von der Brücke (5) abgewandt sind, jeweils mit Haken (11) versehen sind, die zweite innere Abdeckung (26) und die erste innere Abdeckung (25) jeweils fest mit der Unterseite der ersten Abdeckung (3) und der zweiten Abdeckung (4) verbunden sind, der zweite Zylinder (7) abnehmbar mit einer Welle (8) versehen ist, die Wickelfolie und die Welle (8) an der ersten Abdeckung (3) und der zweiten Abdeckung (4) befestigt sind und dadurch eine vollständige Wickelvorrichtung einschließlich der ersten Abdeckung (3) und der zweiten Abdeckung (4) bilden. Der Film, der Schaft (8), die Brücke (5), die erste Innenabdeckung (25), die zweite Innenabdeckung (26) (die nach Verbrauch der Filmrolle ersetzt werden muss) sind **dadurch gekennzeichnet, dass** das Befestigungssystem (17) eine Abdeckplatte (19), eine Schnalle (20), einen Verbindungsstift (21), eine Befestigungsplatte (22) und eine Schale (23) umfasst, wobei die Befestigungsplatte (22) mittels des Verbindungsstifts (21) an der Schutzbrille (1) befestigt ist.

2. Das Schutzfoliensystem nach Anspruch 1, wobei am vorderen Ende des Schafts (8) eine gerade Nut (16) vorgesehen ist, ein Mechanismus (9) am vorderen Ende des zweiten Zylinders (7) beweglich angebracht ist und das vordere Ende des Schafts (8) durch den Mechanismus (9) hindurchgeht und in diesen hineinragt.

3. Das Aufrollsystem für die Schutzbrillenfolie nach Anspruch 2, wobei der Abzieher (10) beweglich mit dem rechten Ende des Mechanismus (9) verbunden ist.

4. Das Brillenverschlusssystem nach Anspruch 1, wobei ein Ende des Riegels (20) durch das Gehäuse (23) hindurchgeht und an dem Verbindungspfosten (21) befestigt ist, und die Abdeckplatte (19) so konfiguriert ist, dass sie an der Unterseite des Gehäuses (23) befestigt werden kann.

5. Das Brillen-Rollfoliensystem nach Anspruch 1, wobei der erste Rollverschluss (6) und der zweite Rollverschluss (7) jeweils mit ihren Führungsnuten (18) versehen sind und die beiden Seiten der zweiten Innenabdeckung (26) und der ersten Innenabdeckung (25) jeweils fest mit den entsprechenden Befestigungsplatten (24) verbunden sind, die mit den entsprechenden Führungsnuten (18) übereinstimmen.

## Revendications

1. Système de film déroulant pour lunettes de protection, comprenant : des lunettes de protection (1) ; et un système déroulant (2) ; dans lequel le système déroulant (2) comprend un premier capuchon (3), un deuxième capuchon (4), une premier cartouche (6), une deuxième cartouche (7) et un système de fixation (17), et le premier cartouche (6) et le deuxième cartouche (7) sont fixés sur les lunettes de protection (1) par le biais du système de fixation (17), et les côtés du premier cartouche (6) et du deuxième cartouche (7) éloignés des lunettes de protection (1) sont pourvus d'une fente de logement (15) respective correspondant au crochet (11) correspondant ; **caractérisé en ce que** le système de déroulant (2) comprend en outre un pont (5), le premier capuchon (3) et le deuxième capuchon (4) étant reliés de manière fixe par le pont (5), les deux extrémités d'un fond du premier capuchon (3) étant respectivement pourvues d'une nervure de fixation (14), les extrémités d'un fond du deuxième capuchon (4) étant respectivement pourvues d'une première rainure de réception (12) et d'une deuxième rainure de réception (13), et comprenant en outre des crochets (11) disposés respectivement sur les fonds des côtés du premier capuchon (3) et du deuxième capuchon (4) éloignés du pont (5), et comprenant en outre un deuxième capuchon intérieur (26) et un premier capuchon intérieur (25) reliés de manière fixe respectivement aux fonds du premier capuchon (3) et du deuxième capuchon (4), le deuxième cartouche (7) est pourvu d'un manche (8) amovible, un film déroulable et la manche (8) étant fixé sur le premier capuchon (3) et du deuxième capuchon (4) afin d'obtenir un ensemble complet d'accessoires comprenant le premier capuchon (3) du deuxième capuchon (4) avec le film déroulant, la manche (8), le pont (5), le premier capuchon intérieur (25), le deuxième capuchon intérieur (26) à remplacer une fois le film déroulant épuisé, et **caractérisé en outre en ce que** le système de fixation (17) comprend une plaque de recouvrement (19), un verrou (20), un montant de connexion (21), une plaque de fixation (22) et un boîtier (23), et la plaque de fixation (22) est fixée sur les lunettes de protection (1) par le biais du montant de connexion (21).

2. Système de film déroulant pour lunettes de protection selon la revendication 1, dans lequel une fente droite (16) est prévue à une extrémité avant de la manche (8), une extrémité avant du deuxième cartouche (7) est montée de manière mobile avec un mécanisme (9), et l'extrémité avant de la manche (8) passe à travers le mécanisme (9) et s'étend à l'intérieur de celui-ci.

3. Système de film déroulant pour lunettes de protection selon la revendication 2, dans lequel un dispositif de tirette (10) est relié de manière mobile à une extrémité droite du mécanisme (9).

4. Système de film déroulant pour lunettes de protection selon la revendication 1, dans lequel une extrémité du verrou (20) pénètre à travers le boîtier (23) pour se fixer au montant de connexion (21), et la plaque de recouvrement (19) est configurée pour se fixer au fond du boîtier (23).

5. Système de film déroulant pour lunettes de protection selon la revendication 1, dans lequel le premier cartouche (6) et le deuxième cartouche (7) sont pourvus d'une fente de guidage (18) respective, les deux côtés du deuxième capuchon intérieur (26) et du premier capuchon intérieur (25) sont reliés de manière fixe à des plaques de maintien (24) respectives correspondant aux fentes de guidage (18) correspondantes.
